# EUROPÄISCHE PATENTANMELDUNG

(11) **EP 2 356 980 A2**
(43) Veröffentlichungstag der Anmeldung: **17.08.2011**
(21) Anmeldenummer: 10007951.6
(22) Anmeldetag: 30.07.2010
(51) Int. Cl.: A61K 8/49, A61Q 19/08, A61K 8/60

(54) **Verwendung von Dihydromyricetin gegen Altershaut**

(30) Priorität: 27.11.2009 DE 102009055916
(71) Anmelder: BEIERSDORF AG, 20245 Hamburg (DE)
(72) Erfinder: Heuser, Stefan, 90762 Fürth (DE); Knott, Anja, 22529 Hamburg (DE); Winnefeld, Marc, 22880 Wedel (DE)
(74) Vertreter: Wilke, Jochen

(57) **Zusammenfassung**

Verwendung von Dihydromyricetin zur Herstellung kosmetischer oder dermatologischer Zubereitungen zur zur Steigerung der dermalen Collagensynthese sowie zur Behandlung und Prophylaxe der Symptome der intrinsischen und/oder extrinsischen Hautalterung.

## Beschreibung

Die vorliegende Erfindung betrifft kosmetische bzw. dermatologische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Die chronologische Hautalterung wird z.B. durch endogene, genetisch determinierte Faktoren verursacht. In Epidermis und Dermis kommt es alterungsbedingt z.B. zu folgenden Strukturschaden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Insbesondere nimmt im Alter das subkutane Fettgewebe, insbesondere in der Peripherie (z.B. im Gesicht) ab, sodaß es zu typischen Altershauterscheinungen kommen kann.

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Insbesondere betrifft die vorliegende Erfindung kosmetische Zubereitungen, die imstande sind, das dermale Bindegewebe der Altershaut zur gesteigerten Collagensynthese zu stimulieren und damit einen zentralen Beitrag zur Behandlung und Prophylaxe der Symptome der intrinsischen und/oder extrinsischen Hautalterung zu leisten.

Aufgabe der vorliegende Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

Diese Aufgabe wird erfindungsgemäß gelöst durch die Verwendung von Dihydromyricetin zur Herstellung kosmetischer oder dermatologischer Zubereitungen zur zur Steigerung der dermalen Collagensynthese sowie zur Behandlung und Prophylaxe der Symptome der intrinsischen und/oder extrinsischen Hautalterung.

Bevorzugt enthalten kosmetische oder dermatologische Zubereitungen gemäß der Erfindung 0,001 - 10 Gew.-%, besonders bevorzugt 0,01 - 1 Gew.-% Dihydromyricetin, bezogen auf die Gesamtzusammensetzung der Zubereitungen.

Dihydramyricetin (oder (+)-Dihydromyricetin; Ampelopsin; (2R,3R)-3,5,7-Trihydroxy-2-(3,4,5-trihydroxyphenyl)chroman-4-on) ist durch folgende Struktur gekennzeichnet:

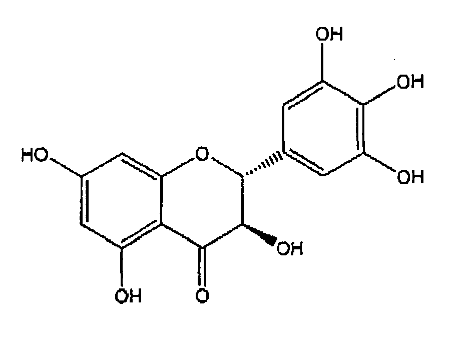

Es ist aus JP 63316711 bekannt, Dihydromyricetin als hautaufhellenden Wirkstoff zu verwenden. Ferner ist aus der FR 2868701 bekannt, Dihydromyricetin als Wirkstoff gegen Cellulite zu verwenden, da dieser die Fettspeicherung in Fettzellen beeinflusst. Dennoch konnte der Stand der Technik nicht den Weg zur vorliegenden Erfindung weisen.

Emulsionen sind vorteilhafte Darreichungsformen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise

Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe lsopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl-oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und mikrokristalline Wachse bzw. Ozokerit) sowie hochschmelzende Wachse (z.B. Carnaubawachs, Candelillawachs)

Als Treibmittel für aus Aerosolbehältem versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Oruckluft ist vorteilhaft zu verwenden.

Die nachfolgenden Beispiele sollen die vorliegende Erfindung verdeutlichen.

Anti-Falten-Creme (Beispiel 1)

| **Beispiel** | **1-1** | **1-2** |
|---|---|---|
| **INCI - Bezeichnung** | **Gew.-%** | **Gew.%** |
| Stearinsäure | 2,50 | 2,50 |
| Glycerylstearat | 1,00 | 1,00 |
| C12-15 Alkylbenzoat | 5,00 | 5,00 |
| Capryl/caprinsäure-Triglycerid | 2,50 | 2,50 |
| Cetylalkohol | 2,00 | 2,00 |
| Stearylalkohol | 2,00 | 2,00 |
| Cyclomethicon | 1,00 | 1,00 |
| Dicaprylylcarbonat | 2,00 | 2,00 |
| Dimethicon | 1,00 | 1,00 |
| Glycerin | 5,00 | 5,00 |
| Methylparaben | 0,20 | - |
| Phenoxyethanol | 0,40 | 0,40 |
| Propylparaben | 0,10 | 0,10 |
| Natrium metabisulfit | - | 0,15 |
| Carbomer | 0,10 | 0,10 |
| Natriumpolyacrylat | - | 0,50 |
| Trinatrium-EDTA + Wasser | 0,2 | 0,20 |
| Tapioka Stärke | 1,50 | 1,50 |
| Diethylhexyl Syringylidenemalonat + Capryl/Caprinsäure-Triglycerid | 0,10 | 0,10 |
| Dihydromyricetin | 0,15 | 0,5 |
| Phenylbenzimidazol Sulfonsäure | 1,00 | 1,00 |
| Natriumhdroxid | q.s. | q.s. |
| Parfum | q.s. | q.s. |
| Wasser | ad 100 | ad 100 |

Anti-Falten-Creme (Beispiel 2)

| **Beispiel** | **2-1** | **2.2** |
|---|---|---|
| **INCI - Bezeichnung** | **Gew.-%** | **Gew.-%** |
| Glycerylstearatcitrat | 2,00 | 3,00 |
| Behenylalkohol | 1,04 | - |
| Glycerylstearat | - | 1,00 |
| C12-15 Alkylbenzoat | 2,50 | 3,00 |
| Capryl/Caprinsäure-Triglycerid | 2,50 | 2,00 |
| Cetylalkohol | 2,00 | 2,00 |
| Cyclomethicon | 2,00 | 2,00 |
| Dicaprylylcarbonat | 2,50 | 2,50 |
| Dimethicon | 1,00 | - |
| Glycerin | 6,00 | 8,00 |
| Methylparaben | 0,05 | 0,20 |
| Phenoxyethanol | 0,40 | 0,40 |
| Propylparaben | 0,10 | 0,10 |
| Natrium metabisulfit | 0,10 | 0,10 |
| Carbomer | 0,10 | 0,20 |
| Natriumpolyacrylat | 0,50 | - |
| Talk | 1,00 | 1,00 |
| Dihydromyricetin | 0,50 | 2,00 |
| Butylmethoxydibenzoylmethan | - | 2,00 |
| Ethylhexylmethoxycinnamat | - | 2,00 |
| Titandioxid | 1,00 | 1,00 |
| Octocrylen | 1,00 | 3,00 |
| Natriumydroxid | q.s. | q.s. |
| Parfum | q.s. | q.s. |
| Wasser | ad 100 | ad 100 |

Anti-Falten-Creme (Beispiel 3)

| **Beispiel** | **3-1** | **3-2** |
|---|---|---|
| **INCI - Bezeichnung** | **Gew.-%** | **Gew.-%** |
| Polyglyceryl-3 methylglucosedistearat | 2,50 | 2,50 |
| Sorbitanstearat | 1,00 | 3,00 |
| C12-15 Alkylbenzoat | 2,50 | 2,50 |
| Capryl/Caprinsäure-Triglycerid | 2,50 | 2,50 |
| Stearylalkohol | 1,00 | 1,50 |
| Cyclomethicon | 3,00 | 1,00 |
| Dicaprylylcarbonat | 2,50 | - |
| Paraffinum Liquidum | - | 1,00 |
| Dimethicon | - | 1,00 |
| Glycerin | 3,00 | 7,50 |
| Methylparaben | - | 0,20 |
| Phenoxyethanol | 0,40 | 0,40 |
| Propylparaben | 0.10 | 0,10 |
| Natrium metabisulfit | 0,20 | - |
| Carbomer | 0,10 | 0,10 |
| Trinatrium-EDTA + Wasser | - | 1,00 |
| Natriumpolyacrylat | 0,30 | - |
| Tapioka Stärke | - | 2,50 |
| Talk | 2,00 | - |
| Dihydromyricetin | 0,05 | 1,00 |
| Natriumhydroxid | q.s. | q.s. |
| Parfum | q.s. | q.s. |
| Ethylhexylmethoxycinnamat | - | 5,00 |
| Butylmethoxydibenzoylmethan | - | 2,00 |
| Wasser | ad 100 | ad 100 |

Anti-Falten-Creme (Beispiel 4)

| **Beispiel** | **4-1** | **4-2** |
|---|---|---|
| **INCI - Bezeichnung** | **Gew.-%** | **Gew.-%** |
| PEG-40 Stearat | 1,00 | 1,00 |
| Glycerylstearat | 3,00 | 3,00 |
| C12-15 Alkylbenzoat | 2,50 | 2,00 |
| Capryl/Caprinsäure-Triglycerid | 2,50 | 3,00 |
| Cetearylalkohol | 3,00 | 3,00 |
| Cyclomethicon | 2,00 | 2,00 |
| Dicaprylylcarbonat | 2,50 | 2,50 |
| Dimethicon | 1,00 | 1,00 |
| Glycerin | 7,50 | 4,00 |
| Methylparaben | 0,20 | 0,20 |
| Phenoxyethanol | 0,40 | 0,40 |
| Propylparaben | 0,10 | - |
| Natrium metabisulfit | - | 0,10 |
| Butylenglykol + Iodpropinylbutylcarbamat | - | 0,10 |
| Carbomer | 0,10 | 0,10 |
| Sodiumpolyacrylat | 0,40 | 0,40 |
| Trinatrium EDTA + Wasser | - | 1,00 |
| BHT | - | 0,05 |
| Butyrospermum Parkii Butter | - | 2,00 |
| Talk | 2,00 | - |
| Butylenglykol | - | 3,00 |
| Dihydromyricetin | 0,10 | 0,25 |
| Natriumhydroxid | q.s. | q.s. |
| Parfum | q.s. | q.s. |
| Ethylhexylmethoxycinnamat | 3,00 | - |
| Butylmethoxydibenzoylmethan | 2,00 | - |
| Wasser | ad 100 | ad 100 |

Anti-Falten-Creme (Beispiel 5)

| **Beispiel** | **5-1** | **5-2** |
|---|---|---|
| **INCI-Bezeichnung** | **Gew.-%** | **Gew.-%** |
| PEG-40 Stearat | 1,00 | 1,00 |
| Capryl/Caprinsäure-Triglycerid | 2,50 | 2,50 |
| Octyldodecanol | 2,50 | 2,50 |
| Cetearyl Alkohol | 3,00 | 3,00 |
| Polysorbat 40 | 3,00 | 3,00 |
| Glycerin | 7,00 | 5,00 |
| C12-15 Alkyl Benzoat | 2,50 | 2,50 |
| Dimethicon | 0,35 | 0,35 |
| Cyclomethicon | 2,15 | 2,15 |
| Phenoxyethanol | 0,20 | 0,30 |
| Propylparaben | 0,10 | 0,05 |
| Methylparaben | 0,20 | 0,20 |
| Trinatrium EDTA+ Wasser | 1,00 | 1,00 |
| Dihydromyricetin | 0,15 | 0,15 |
| Diethylhexyl Syringylidenemalonat + Capryl/Caprinsäure-Triglycerid | 0,10 | 0,10 |
| Natrium metabisulfit | - | 0,15 |
| Xanthangummi | 0,10 | 0,10 |
| Natrium Polyacrylate | 0,30 | 0,30 |
| Distärkephosphat | - | 0,10 |
| Octocrylen | 0,10 | - |
| Wasser | Ad 100 | Ad 100 |

Hydrodispersionsgel (Beispiel 6)

| **INCI-Bezeichnung** | **Gew.-%** |
|---|---|
| Alcohol Denat. | 8,00 |
| Natriummetabisulfit | 0,15 |
| Phenoxyethanol | 0,20 |
| Butylenglykol + lodpropinylbutylcarbamat | 0,15 |
| Methylpropanediol | 2,00 |
| Glycerin | 5,00 |
| Butylenglykol | 3,00 |
| Cyclomethicon | 5,00 |
| Cyclomethicon + Dimethiconol | 1,00 |
| Dihydromyricetin | 0,20 |
| Carbomer | 0,20 |
| Natriumpolyacrylat | 0,50 |
| Chondrus Crispus | 0,15 |
| Acrylat/C10-30 Alkylacrylatcrosspolymer | 0,25 |
| Xanthangummi | 0,10 |
| Natriumhydroxid | q.s. |
| Parfum | q.s. |
| Wasser | Ad 100 |

## Patentansprüche

1. Verwendung von Dihydromyricetin zur Herstellung kosmetischer oder dermatologischer Zubereitungen zur zur Steigerung der dermalen Collagensynthese sowie zur Behandlung und Prophylaxe der Symptome der intrinsischen und/oder extrinsischen Hautalterung.

2. Verwendung nach Anspruch 1 in kosmetischen oder dermatologischen Zubereitungen, enthaltend 0,001 - 10 Gew.-% Dihydromyricetin, bezogen auf das Gesamtgewicht der Zubereitungen.
